# EUROPEAN PATENT APPLICATION

(11) **EP 1 072 229 A2**
(43) Date of publication of application: **31.01.2001**
(21) Application number: 00114833.7
(22) Date of filing: 11.07.2000
(51) Int. Cl.: A61B 17/74

(54) **Synthesis element for repairing fractures of the femur**

(30) Priority: 30.07.1999 IT UD990047 U
(71) Applicant: LIMA Lto SpA, 33030 Villanova di S. Daniele (UD) (IT)
(72) Inventor: Lualdi, Gabriele, 33034 Fagagna (UD) (IT)
(74) Representative: Petraz, Gilberto Luigi

(57) **Abstract**

Synthesis element (10) for repairing fractures of the femur comprising a femoral component able to be associated with the femur, and a cephalic screw (11) able to couple said femoral component to the femur head, said cephalic screw (11) comprising first association means to couple a first femoral component comprising a rod (12) able to be inserted into a diaphysis canal made in the femur and second association means to couple a second femoral component comprising a plate (13) able to be laterally attached to the outer surface of the femur, said first and second association means being able to be used alternately to define at least two distinct configurations of use for the synthesis element (10).

## Description

### FIELD OF THE INVENTION

This invention concerns a synthesis element for repairing fractures of the femur able to be used in the field of traumatology, and particularly in the field of bone synthesis following a traumatic lesion.

In the field of traumatology, the term "synthesis" means the joint or combination resulting from setting parts separated after a traumatic lesion.

### BACKGROUND OF THE INVENTION

The state of the art includes synthesis elements used in the field of traumatology to synthesize fractures of the femur.

Such synthesis elements comprise a femoral component able to be coupled with the femur and a cephalic screw able to be coupled at one end to said femoral component and at the opposite end with the femur head.

At present, two different types of synthesis elements are used, one instead of the other according to the type of fracture and/or the morphology of the femur.

In the first type, the synthesis element uses a femoral component consisting of a rod able to be inserted and attached inside the diaphysis canal of the femur.

In the second type, employed when it is not possible to use the diaphysis canal of the femur or whenever the type of fracture requires it, the synthesis element uses a femoral component consisting of a holed plate able to be attached on the lateral outer surface of the femur.

At present therefore, according to the type of surgical operation, it is necessary to have available both types of synthesis element since, usually, it is only during the operation itself that it is possible to know which type is more suitable.

This is a problem since having two different types of element available causes problems in management and of a practical nature.

Moreover, especially in the second type of synthesis element, regulating the inclination of the holed plate with respect to the cephalic screw, which is carried out during the surgical operation according to the morphology of the articulation, is not very accurate and reliable.

The present Applicant has devised and embodied this invention to overcome these shortcomings, and to obtain other advantages.

### SUMMARY OF THE INVENTION

The invention is set forth and characterized in the main claim, while the dependent claims describe other characteristics of the invention.

The purpose of the invention is to provide a single bone synthesis element for proximal fractures of the femur, able to assume selectively two different configurations of use: a first configuration wherein the femoral component consists of a rod able to be inserted into a diaphysis canal made in the femur and a second configuration wherein the femoral component consists of a holed plate able to be laterally associated with the outer surface of the femur.

The synthesis element according to the invention, being suitable to assume the two configurations normally carried out by two different types of conventional synthesis elements, is characterized in that it has smaller management costs and is more practical in use.

Another purpose of the invention is to achieve a synthesis element which will allow to regulate the femoral component easily and precisely with respect to the cephalic screw.

In accordance with these purposes, a synthesis element according to the invention comprises, in its essential parts, a single cephalic screw and a replaceable femoral component.

According to the main characteristic of the invention, the cephalic screw is integrally equipped with first coupling means able to retain a first femoral component consisting of a rod, and second coupling means able to retain a second femoral component consisting of a holed plate, said first and second femoral components being used in alternation.

In the preferential embodiment of the invention, said first and said second coupling means have parts and/or components in common.

In one embodiment of the invention the coupling means comprise a threaded axial hole made on the cephalic screw in correspondence with one of its ends and a transverse and inclined through hole made on the body of the cephalic screw, said through hole and said threaded axial hole communicating with each other.

In this embodiment, the first configuration is obtained by inserting the rod into the transverse through hole; the rod is clamped in a desired position by means of a screw able to be inserted and tightened in the threaded axial hole.

The second configuration, instead, is obtained by inserting into a through hole made on the upper end of the plate a screw able to be inserted and tightened in the threaded axial hole.

According to a variant, between the plate and the coupling end of the cephalic screw a positioning element is interposed, which allows to vary the angular position of the plate with respect to the cephalic screw.

### BRIEF DESCRIPTION OF THE DRAWINGS

These and other characteristics of the invention will be clear from the following description of a preferred form of embodiment, given as a non-restrictive example with reference to the attached Figures wherein:
- Fig. 1: is an exploded view of some components of the synthesis element according to the invention for repairing fractures of the femur;
- Fig. 2: shows the synthesis element as in Fig. 1 in a first configuration of use;
- Fig. 3: shows the synthesis element as in Fig. 1 in a second configuration of use.

### DETAILED DESCRIPTION OF PREFERRED EMBODIMENT

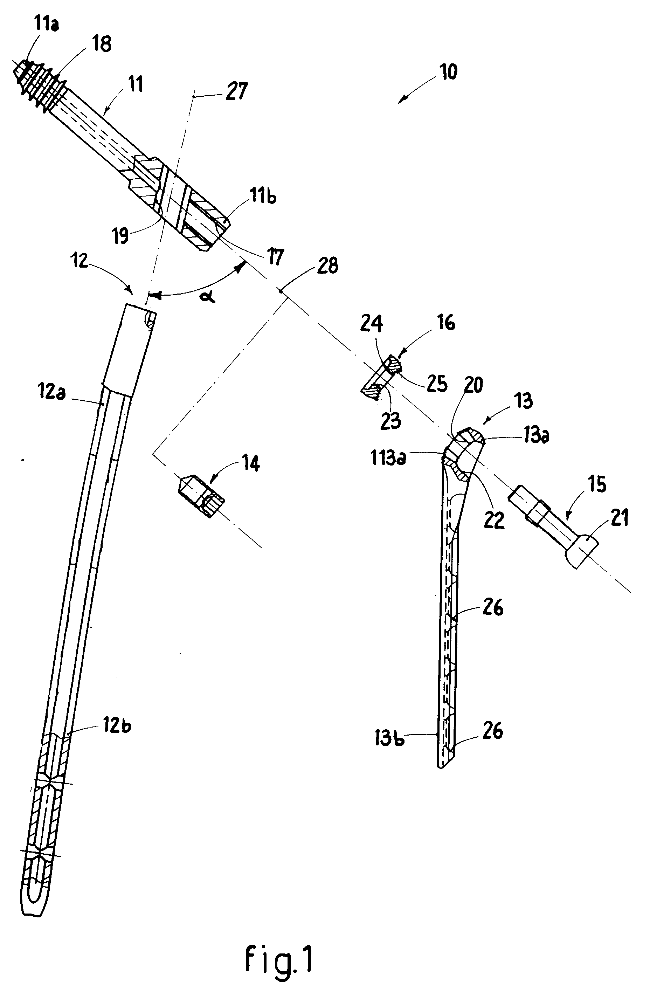

Fig. 1 is a part view of a synthesis element 10 according to the invention which comprises a cephalic screw 11, a rod 12, a plate 13, a first attachment screw 14, a second attachment screw 15 and a positioning crown 16.

The cephalic screw 11 is equipped on the front end 11a with a thread 18 to constrain it to the head of the femur, on the rear end 11b with a threaded axial hole 17 and in an intermediate position with a transverse through hole 19 made on the body at a defined distance from said rear end 11b.

The transverse through hole 19 has its axis 27 inclined by a defined angle α with respect to the longitudinal axis 28 of the cephalic screw 11 and communicates with the threaded axial hole 17.

According to the invention, the first screw 14, the transverse through hole 19 and the threaded hole 17 define first association means able to allow, in a first use, the coupling of the rod 12 with the cephalic screw 11, whereas the second screw 15, the positioning crown 16 and the threaded hole 17 define second association means which allow, in a second and alternative use, the coupling of the plate 13 with the cephalic screw 11.

The rod 12 has an upper part 12a able to be coupled with the cephalic screw 11 and a lower part 12b able to be inserted into the diaphysis canal of the femur.

The plate 13 has an upper part 13a able to be coupled with the cephalic screw 11 and equipped for this purpose with a through hole 20, and a lower part 13b equipped with a plurality of holes 26 into which respective screws, not shown here, are able to be inserted, to constrain the plate 13 to the femur.

The first attachment screw 14 is of the headless type with a sunken hexagonal socket, whereas the second attachment screw 15 has a hemispheric head 21 able to be inserted into a mating hemispheric seating 22 made on the plate 13 in correspondence with the through hole 20.

The positioning crown 16 is axially equipped with a through hole 23 and has a cavity 24 on a first side into which the rear end 11b of the cephalic screw 11 is able to be inserted and on the opposite side a cavity 25 into which a mating portion 113a of the upper part 13a is able to be inserted.

To be more exact, both said cavity 25 and said portion 113a are equipped with knurls, in this case of the type with parallel lines, which allow to regulate the angular position of the plate 13 with respect to the positioning crown 16 with extreme accuracy and reliability and, consequently, with respect to the cephalic screw 11.

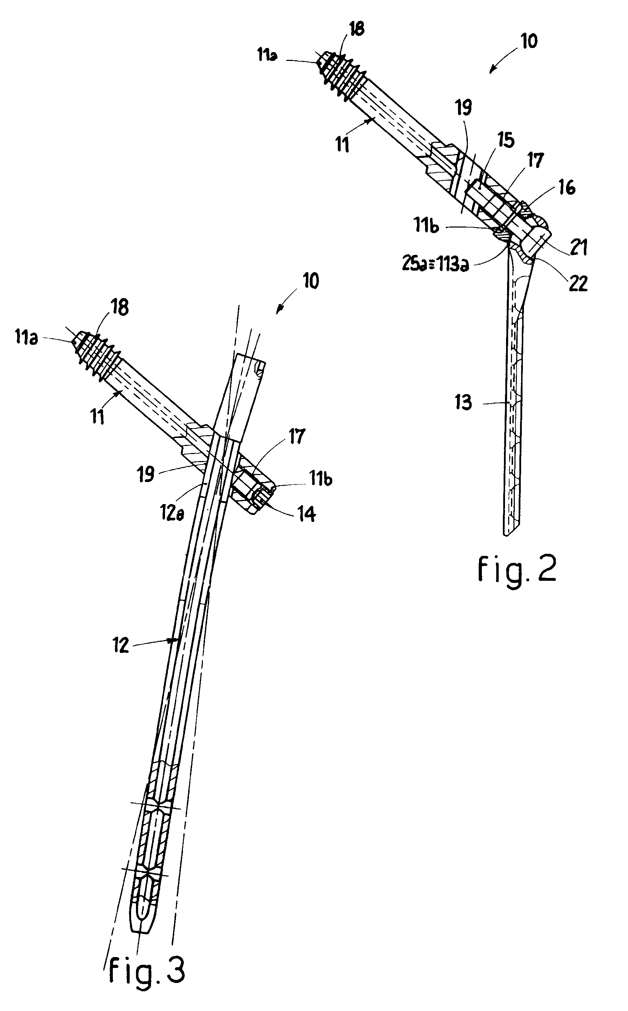

The synthesis element 10 according to the invention, therefore, can selectively assume a first configuration, shown in Fig. 3, able to be used in cases when, during the operation, it is possible or necessary to use the diaphysis canal of the femur, or a second configuration, shown in Fig. 2, able to be used in other cases.

In the first configuration, the rod 12 is inserted into the transverse through hole 19 and clamped therein in the desired position by tightening the first attachment screw 14 into the threaded axial hole 17.

In the first configuration, obviously, the plate 13, the second attachment screw 15 and the positioning crown 16 are not used.

In the second configuration, the second clamping screw 15 is inserted into the through hole 20 of the plate 13 and into the through hole 23 of the positioning crown 16 and then tightened into the threaded axial hole 17 of the cephalic screw 11.

In this configuration, obviously, the rod 12 and the first attachment screw 14 are not used.

It is obvious that modifications and additions may be made to the synthesis element 10 as described heretofore, but these shall remain within the field and scope of the invention. Likewise, although the description refers to a specific example, a person of skill shall certainly be able to achieve many other equivalent forms of synthesis elements, but they too shall all come within the field and scope of this invention.

## Claims

1. Synthesis element for repairing fractures of the femur comprising a femoral component able to be associated with the femur, and a cephalic screw able to couple said femoral component to the femur head, the synthesis element being characterized in that said cephalic screw (11) comprises first association means to couple a first femoral component comprising a rod (12) able to be inserted into a diaphysis canal made in the femur and second association means to couple a second femoral component comprising a plate (13) able to be laterally attached to the outer surface of the femur, said first and second association means being able to be used alternately to define at least two distinct configurations of use for the synthesis element (10).

2. Synthesis element as in Claim 1, characterized in that said first association means and said second association means have parts or components in common.

3. Synthesis element as in Claim 1 or 2, characterized in that said first association means comprise a transverse inclined through hole (19) made on the body of said cephalic screw (11) and a threaded axial hole (17) made on one end (11b) of said cephalic screw (11) and communicating with said through hole (19), said rod (12) being inserted into said through hole (19) and clamping means (14) to clamp said rod (12) being inserted into said threaded axial hole (17).

4. Synthesis element as in Claim 1 or 2, characterized in that said second association means comprise a threaded axial hole (17) made on one association end (11b) of said cephalic screw (11), clamping means (15) being able to be inserted into a through hole (20) made on said plate (13) and into said threaded axial hole (17) to constrain said plate (13) to said cephalic screw (11).

5. Synthesis element as in Claim 4, characterized in that between said plate (13) and said association end (11b) a positioning element is provided able to allow, during the assembly stage, variations in the angular position of said plate (13) with respect to said cephalic screw (11).

6. Synthesis element as in Claim 5, characterized in that said positioning element comprises a crown (16) equipped with an axial through hole (23) into which said clamping means (15) are able to be inserted and, on the coupling side with said plate (13), with knurls able to cooperate with mating knurls on said plate (13) to define different angular positions of said plate (13) with respect to said cephalic screw (11).

7. Synthesis element as in Claim 4, characterized in that said clamping means (15) are equipped with a hemispheric head (21) able to be inserted into a mating hemispheric cavity (22) made in correspondence with said through hole (20).
